# EUROPÄISCHE PATENTSCHRIFT

(11) **EP 2 512 270 B1**
(45) Veröffentlichungstag und Bekanntmachung des Hinweises auf die Patenterteilung: **13.11.2013**
(21) Anmeldenummer: 11831817.9
(22) Anmeldetag: 20.12.2011
(51) Int. Cl.: A23L 2/52, A23L 2/60, A23L 2/68

(54) **ERFRISCHUNGSGETRÄNK**
REFRESHING BEVERAGE
BOISSON RAFRAÎCHISSANTE

(30) Priorität: 06.01.2011 DE 102011008017
(43) Veröffentlichungstag der Anmeldung: 24.10.2012
(73) Patentinhaber: Coy, Johannes, 64853 Otzberg (DE)
(72) Erfinder: Coy, Johannes, 64853 Otzberg (DE)
(74) Vertreter: Rudolph, Ulrike
(86) Internationale Anmeldenummer: PCT/DE2011/002144
(87) Internationale Veröffentlichungsnummer: WO 2012/092917

(56) Entgegenhaltungen:
- WO-A1-2008/112857
- JP-A- 7 277 991
- US-A1- 2008 292 765

## Beschreibung

Die Erfindung betrifft ein neues phosphorsäurefreies, mineralarmes, zuckerarmes, süßschmeckendes Erfrischungsgetränk.

Das Gefühl Durst entsteht entweder, wenn im Körper ein Flüssigkeitsmangel auftritt oder wenn ein Salzüberschuss (Mineralüberschuss) vorliegt. Durch Flüssigkeitsaufnahme kann der Flüssigkeitsmangel oder der Mineralienüberschuss im Körper ausgeglichen werden, vorausgesetzt die Flüssigkeit ist mineralienfrei oder mineralienarm. Ein hoher Mineralgehalt der Flüssigkeit, wie im Fall von Meerwasser kann dagegen zu der paradoxen Situation führen, dass ein Mensch trotz Flüssigkeitsaufnahme verdurstet, weil der hohe Mineralgehalt in dem Meerwasser keinen Netto-Abtransport von Mineralien aus dem Körper mehr möglich macht. Trotz Trinkens steigt der Mineralüberschuss im Körper noch an, bis schließlich der Tod eintritt. Deshalb sollte eine Trinkflüssigkeit wenig Mineralien enthalten, wenn nicht eine Erhöhung des Mineralgehaltes im Körper erwünscht ist.

Die Bevölkerung der westlichen Industrienationen hat großenteils eine Lebensweise mit relativ wenig körperlicher Bewegung. Situationen, in denen der Körper schwitzt und infolgedessen ein hoher Mineralienverlust auftritt, sind bei den Menschen dieser Bevölkerungsgruppen gewöhnlich die Ausnahme. Deshalb sollten gerade diese Menschen ihren täglichen Flüssigkeitsbedarf vorrangig oder vollständig mit Getränken decken, die mineralienarm sind.

Der übliche Getränkekonsum der Menschen in den westlichen Industrienationen führt aufgrund des Überangebotes von süßen Getränken bei vielen Menschen und insbesondere bei vielen Kindern zu einer Überversorgung des menschlichen Körpers mit Zucker in Form von Glukose und Fruktose. Die meisten handelsüblichen herkömmlichen Getränke weisen einen relativ hohen Gehalt an Saccharose (Rohrzucker) auf, einem Disaccharid aus Glukose und Fruktose. Saccharose besitzt einen hohen glykämischen Index, d.h. die Aufnahme von Saccharose in den Körper führt zu einem schnellen und starken Anstieg des Blutzuckerspiegels und infolgedessen auch zu einem starken Anstieg des Insulinspiegels im Blut.

Der Konsum von saccharosereichen Getränken (Zuckergehalt mehr als 4% Gew./Vol) führt zu einem besonders schnellen und hohen Blutzuckeranstieg und hat damit eine sehr hohe Insulinausschüttung zur Folge. Die Wirkung des Insulins führt nach dem Prinzip der negativen Rückkopplung wieder zu einem Abfall des Blutzuckerspiegels, und bei einem hohen Insulinspiegel erfolgt dieser Abfall entsprechend drastisch. Der drastische Abfall des Blutzuckerspiegels führt dann wiederum zu einem Leistungsabfall und Konzentrationsschwäche und löst im Gehirn das Gefühl des Heißhungers auf Süßes aus, damit durch Aufnahme von Zuckern der Blutzuckerspiegel wieder angehoben wird. Der betreffende Konsument befindet sich folglich in einer Bedürfnis-Spirale, die dazu verleitet, immer wieder zuckerreiche und damit kalorienreiche Getränke zu konsumieren.

Darüber hinaus müssen bzw. sollten Zucker mit hohem glykämischen Index insbesondere von Diabetes-Typ-2-Patienten und von Krebs-Patienten gemieden werden.

Die aktuelle Zunahme der Inzidenz von Diabetes Typ 2 unterstreicht die Notwendigkeit der Bereitstellung von Nahrungsmitteln inklusive Getränken, die einen relativ geringen glykämischen Index (GI) und eine relativ geringe glykämische Last (GL) haben und deshalb einen nur einen relativ niedrigen Insulinausstoß bewirken.

Die Erkrankung Krebs ist bei Diabetikern häufiger als im Durchschnitt der Bevölkerung. Die aktuell hohe Kohlenhydrataufnahme in der Bevölkerung der westlichen Industrienationen und der zunehmende Kohlenhydratkonsum in Ländern wie Indien und China führen nicht nur zu einer weiter steigenden Inzidenz von Diabetes, sondern auch von Krebs.

Krebs ist ein mehrstufiger Prozess, der aus einer gesunden Zelle über eine gutartige Tumorzelle zur Entstehung einer bösartigen Tumorzelle (Krebszelle) führt. Auslöser dieses Prozesses sind Genveränderungen (Mutationen), die die Wachstums- und Absterbeeigenschaften einer Zelle so verändern, dass diese sich teilt und damit vermehrt, ohne dass dies dem Gesamtorganismus nutzt. Durch die gesteigerte Zellvermehrung und das verminderte Absterben der Zellen entsteht zunächst eine Zellansammlung/Zellhaufen, die/der als gutartiger Tumor bezeichnet wird. Der gutartige Tumor verdrängt das umliegende gesunde Gewebe, ohne es zu zerstören oder darin hineinzuwachsen (nichtinvasives Wachstum). Die gutartigen Tumore können sich zu bösartigen Tumoren entwickeln, indem sie ihren Stoffwechsel verändern, nämlich von Verbrennungsstoffwechsel auf Vergärungsstoffwechsel umschalten, und zwar auch in Anwesenheit von Sauerstoff (aerobe Glykolyse oder Warburg-Effekt). Es sind mehrere Einflüsse bekannt, die diese Umschaltung auslösen: Sauerstoffarmut in größeren oder schlecht durchbluteten Tumoren, Radikalbelastung der Tumorzelle in Folge von chronischen Entzündungen, Chemo- und Strahlentherapien und Anti-Angiogenesewirkstoffe. Die beim Vergärungsstoffwechsel gebildete Milchsäure zerstört das umliegende Gewebe, so dass der Tumor dort hinein wachsen kann (invasives Wachstum), und sie hemmt den Angriff des Immunsystems. Die nun invasiv wachsenden Zellen können sich über das Lymph- und Blutgefäßsystem im ganzen Körper ausbreiten und Femmetastasen bilden (Streuung). Durch die Abschaltung des Verbrennungsstoffwechsels in den Mitochondrien (oxidative Phosphorylierung) wird die Bildung von Radikalen und die Auslösung von Apoptose unterdrückt, und hierdurch werden diese Krebszellen auch resistent gegenüber Strahlen- und Chemotherapien.

Ein erhöhter Konsum von leicht verdaulichen Kohlenhydraten (insbesondere in Form von Zuckern und Stärke mit hohem glykämischen Index) begünstigt den Übergang von Tumorzellen in Krebszellen und damit einhergehend eine Aktivitätssteigerung des Gens TKTL1 (Transketolase-like-1 Gen). Die TKTL1-Genprodukte bewirken eine sauerstoffunabhängige Energiefreisetzung, die zu keiner Radikalbildung führt und vorhandene oder exogen induzierte Radikale neutralisiert. Gleichzeitig wird die Aktivität der Mitochondrien reduziert und die Apoptose (programmierter Zelltod) wird gehemmt. Nach dem derzeitigen Stand der Wissenschaft ist eine erhöhte Aktivität des TKTL1-Gens ursächlich oder zumindest ein wesentlicher Grund für die Entstehung von aggressiven Krebserkrankungen.

Mit dem Nachweis der Aktivität des TKTL1-Gens in Tumorzellen (z.B. direkt in Tumorzellen oder indirekt durch Nachweis des TKTL1-Proteins in Körperflüssigkeiten oder in Fresszellen/Makrophagen) können Krebspatienten identifiziert werden, denen eine Ernährungstherapie mit Beschränkung der Kohlenhydratmenge und Verwendung von Zuckerformen mit niedrigem glykämischen Index Linderung oder gar Heilung bringen kann, weil das Wachstum von TKTL1-positiven Tumoren und Metastasen infolge der reduzierten Kohlenhydrat- und insbesondere Glukosezufuhr gehemmt wird.

Da oftmals auch die Zuckeraufnahme in Krebszellen mit Hilfe von Insulin erleichtert wird und Insulin zudem eine wachstumsfördernde Wirkung auch auf Krebszellen ausübt, ist es grundsätzlich wünschenswert, nach dem Verzehr einer Mahlzeit nur einen geringen Insulinausstoß zu induzieren.

Neben den süßen Getränken, die Rohrzucker/Saccharose als Zuckerkomponente enthalten, gibt es auch solche, bei denen die Zuckerkomponente ganz oder größtenteils in Form von Zuckeralkoholen, künstlichen Süßstoffen, Fruktose, Isomaltulose oder Kombinationen davon realisiert ist. So werden z.B. in den sogenannten Diabetikergetränken anstelle von Saccharose entweder Zuckeralkohole (wie z.B. Polyole) oder Fruktose oder künstliche Süßstoffe oder Kombinationen davon eingesetzt.

Die Zuckeralkohole weisen einen niedrigen glykämischen Index auf, doch können sie zu Gesundheitsproblemen führen, weil sie von menschlichen Enzymen nicht vollständig verdaut werden können und teilweise über die Darmflora abgebaut werden, was oft zu Gasbildung, Blähungen und Durchfällen führt.

Künstliche Süßstoffe weisen ebenfalls einen niedrigen glykämischen Index auf, für viele von ihnen sind aber mittlerweile zum Teil gravierende gesundheitsschädigende Nebenwirkungen bekannt. So ist für Aspartam nachgewiesen, dass es nach längerer Lagerung oder Lagerung in warmer Umgebung in den Alkohol Methanol übergeht, der sich zu Formaldehyd und Formsäure, zwei krebserregende Stoffe, umwandeln kann. Der künstliche Süßstoff Saccharin gilt sowohl in der human- als auch der veterinärmedizinischen Forschung als Mitverursacher von Blasenkrebs, in der Veterinärmedizin wird er außerdem noch mit weiteren Krebsformen in Verbindung gebracht.

Auch Fruktose weist einen niedrigen glykämischen Index auf. In der Nahrung des Menschen hat Fruktose jedoch gegenüber Glukose vor allem den Nachteil, dass etwa 30-40% der Bevölkerung in der westlichen Welt eine Fruktose-Malabsorption aufweisen. Außerdem wird Fruktose im Unterscheid zu Glukose unreguliert, allein aufgrund ihres Konzentrationsgradienten vom Darm in die Zellen transportiert. Bei hohen Fruktosemengen in der Nahrung können deshalb gesundheitliche Probleme auftreten wie beispielsweise eine osmotische Diarrhoe, Serotoninmangel (infolge chemischer Reaktionen von Fruktose mit Tryptophan im Darm) oder erhöhte Harnsäureproduktion (infolge erhöhter Fruktose-1-Phosphat-Bildung in der Leber).

Isomaltulose ist ein in Honig und Zuckerrohr vorkommender Zucker, der auch aus Saccharose hergestellt werden kann. Isomaltulose besteht wie Saccharose aus den beiden Monosacchariden Glukose und Fruktose, weist aber ein zwar ähnliches aber reduziertes Süßprofil auf. Aufgrund einer im Vergleich zu Saccharose stabileren Molekülbindung zwischen dem Glukose- und Fruktosemolekül wird Isomaltulose von den menschlichen Disaccharidasen im Dünndarm langsamer gespalten als Saccharose, und damit erfolgt auch der Blutglukose- und Insulinanstieg nur langsam. Der glykämische Index von Isomaltulose ist mit GI=32 deutlich geringer als der von Saccharose (GI=65).

In einigen herkömmlichen Getränken ist Phosphorsäure enthalten, um einen angenehm leicht säuerlichen Geschmack zu erzeugen. Phosphorsäure greift jedoch die Zähne an, und der in ihr enthaltene Phosphor beeinträchtigt im Körper die Aufnahme von Kalzium, was zu einer Schwächung der Knochen führen kann. Für die Framingham-Osteoporose-Studie haben Tucker und ihre Mitarbeiter Untersuchungen an mehr als 2.500 Frauen im Alter unter Sechzig durchgeführt. Sie nahmen dafür Knochendichtemessungen an der Wirbelsäule und drei verschiedenen Hüftregionen vor und stellten fest, dass sich die Knochendichte bei regelmäßig Cola trinkenden Frauen in allen drei Hüftregionen um 4% verringert hatte, unabhängig davon, wie alt sie waren, ob sie sich in der Menopause befanden, zusätzliche Kalzium- oder Vitamin-D-Präparate nahmen oder Alkohol und Zigaretten konsumierten. ("Colas, but not other carbonated beverages, are associated with low bone mineral density in older women: The Framingham Osteoporosis Study" American Journal of Clinical Nutrition, Vol. 84, No. 4, 936-942, October 2006)

Aufgabe der vorliegenden Erfindung ist deshalb die Bereitstellung eines phosphorsäurefreien, mineralien- und zuckerarmen aber dennoch süßschmeckenden Getränks mit niedrigem glykämischem Index, das dazu geeignet ist, einen Flüssigkeitsmangel im Körper auszugleichen, ohne dem Körper dabei hohe Mengen an Mineralien zuzuführen, und das das Genussbedürfnis nach süßen und erfrischenden Getränken erfüllt, ohne dem Körper dabei schädliche Säuren und Zucker in einer Form zuzuführen, die einen steilen Anstieg der Insulinkonzentration im Blut bewirkt, und die im negativsten Fall zu einer insulinbedingten Unterzuckerung und einem Energiemangelzustand nach dem Konsum des Getränkes führen kann.

Eine Lösung dieser Aufgabe besteht in einem phosphorsäurefreien, mineralarmen, zuckerarmen, süßschmeckenden Erfrischungsgetränk, das dadurch gekennzeichnet ist, dass
- es einen Gehalt an Gluconsäure aufweist, der mindestens 0,3 % Gew./Vol. beträgt,
- dass ein etwaiger Gehalt an Mineralien weniger als 250 mg/Liter (0,025 %Gew./Vol.), vorzugsweise weniger als 150 mg Mineralien pro 1 Liter (0,015 %Gew./Vol.) und besonders bevorzugt weniger als 100 mg Mineralien pro 1 Liter (0,01 %Gew./Vol.) beträgt,
- der Gehalt an Zuckern maximal 2,5 % Gew./Vol. beträgt und einen Anteil Galaktose umfasst, der mindestens 10 % Gew./Gew. bezogen auf den Gehalt an Zuckern beträgt, sowie einen Anteil Isomaltulose und/oder Tagatose und/oder Trehalose und/oder Ribose umfasst,
- und dass das Getränk einen glykämischen Index von maximal 35 (GI<35) aufweist.

Mit diesem Erfrischungsgetränk ist es erstmals möglich, den Bedarf des Körpers nach Flüssigkeit zu decken, ohne den bestehenden Mineralhaushalt zu stören, dabei gleichzeitig das Geschmacksbedürfnis nach einem süßen und erfrischenden Getränk zu erfüllen, ohne den bestehenden Blutzuckerspiegel derart zu verändern, dass es zu einem Anstieg der Insulinkonzentration im Blut kommt, und ohne die Zähne einem zahnschädlichen Milieu auszusetzen, und das alles ohne den Einsatz gesundheitsschädlicher Zutaten und ohne den Einsatz von künstlichen Süßstoffen, künstlichen Farbstoffen und künstlichen Aromen. Darüber hinaus bietet das erfindungsgemäße Getränk insbesondere für Menschen, die sich wenig körperlich bewegen, dabei aber geistige Arbeit leisten und Konzentration aufbringen müssen, den Vorteil, dass die enthaltene Galaktose die Nervenzellen mit der notwendigen Energie versorgt, selbst wenn der Glukosespiegel im Blut niedrig ist. Auch für Menschen mit Insulinresistenz ist diese Eigenschaft des Getränks besonders von Vorteil.

Das Getränk eignet sich deshalb erfindungsgemäß auch sehr gut als Nahrungsmittel oder Nahrungsergänzungsmittel in der präventiven oder therapeutischen Behandlung von Krankheiten aus der Gruppe umfassend: Zöliakie, Diabetes mellitus Typ-2, neurodegenerative Krankheiten, insbesondere Alzheimer, Tumorerkrankungen, sowie Erkrankungen, die zu einer Kachexie (Auszehrung) führen, die mit entzündlichen Prozessen einhergehen oder davon verursacht werden, insbesondere Rheuma, rheumatoide Arthritis, entzündliche Darmerkrankungen wie Ulzerative Colitis, Morbus Crohn, Darmerkrankungen wie das Leaky-Gut-Syndrom (durchlässiger Darm).

Mit der erfindungsgemäßen Verwendung kann eine therapeutische oder präventive Behandlung von Personen erfolgen, die entweder von einer genannten Erkrankungen betroffen sind, oder aber die - entweder genetisch oder ernährungstechnisch bedingt - das Risiko tragen, solche Krankheiten zu entwickeln.

Sofern nicht anders angegeben gelten in der vorliegenden Beschreibung und den Ansprüchen folgende Definitionen:
Erfrischungsgetränk = Getränk, das Wasser (Trinkwasser und/oder natürliches Mineralwasser und/oder Quellwasser und/oder Tafelwasser) und geschmacksgebende Zutaten enthält, und darüber hinaus weitere Zusätze enthalten kann, beispielsweise Kohlensäure, Mineralstoffe, Vitamine, Zuckerarten, Fruchtsäfte und/oder Fruchtkonzentrate, Aromen (siehe auch Deutsches Lebensmittelgesetzt, Leitsätze für Erfrischungsgetränke, Fassung 18. März 2003; GMBl. Nr. 18 S. 383)
Zucker = süß schmeckende Mono-, Di- und Trisaccharide wie z.B. Saccharose, Glukose, Laktose, Galaktose, Fruktose, Invertzucker, Isomaltulose, Maltose, Melezitose, Tagatose, Trehalose, Ribose.
Zuckeralkohole (Synonym: Alditole) = nichtzyklische Polyole, die sich strukturell durch Reduktion von Zuckern ableiten, wie z.B. Sorbit, Xylit, Mannit, Maltit, Isomalt, Erythritol.
Zuckerkulör = Lebensmittelfarbe mit süßlichem bis leicht bitterem Aroma, die aus Zuckern, vorzugsweise aus Zuckern mit niedrigem glykämischen Index wie z.B. Isomaltulose, durch Erhitzen auf 120 bis 150 °C und ohne Zusätze von gesundheitsschädlichen Reaktionsbeschleunigern hergestellt wird.
Mineralien = Summe der gelösten mineralischen Bestandteile, insbesondere Magnesium und Calcium, aber auch Natrium, Sulfat, Kalium, Kieselsäure, Chlorid, Fluorid, ausgenommen Hydrogencarbonat und gelöstes Kohlendioxid.
Gehalt an Tocotrienolen = der Gehalt an alpha- und/oder beta- und/oder gamma- und/oder delta-Tocotrienol.
Zuckerarm = nicht mehr als 2,5g Zucker pro 100ml Flüssigkeit (gemäß Berichtigung der Verordnung (EG) Nr. 1924/2006 des Europäischen Parlaments und des Rates vom 20. Dezember 2006 über nährwert- und gesundheitsbezogene Angaben über Lebensmittel - Amtsblatt der Europäischen Union L 12 vom 18. Januar 2007).
Mineralarm = mit geringem Gehalt an Mineralien gemäß deutscher "Mineral- und Tafelwasser-Verordnung vom 1. August 1984 - BGBl. I S. 1036, zuletzt geändert durch Artikel 1 der Verordnung vom 1. Dezember 2006 - BGBl. I S. 2762, zuletzt geändert durch Art. 1 V v. 1.12.2006 BGBl. I S. 2762). Der als fester Rückstand berechnete Mineralstoffgehalt beträgt nicht mehr als 500 mg/l Flüssigkeit.

Galaktose ist ein Zucker, der als Bestandteil des Milchzuckers (Laktose) ein natürlicher Nahrungsbestandteil des Menschen ist. Neben Glukose und Fruktose gehört Galaktose zu denjenigen Monosacchariden, die vom Menschen am meisten konsumiert und damit dem menschlichen Stoffwechsel am meisten zugeführt werden. Galaktose wird als Baustein von Kohlenhydraten in verschiedenen Schleimhäuten benötigt (anabole Reaktionen) und kann bei Bedarf nach Umbau zu Glukose auch für katabole Reaktionen genutzt werden.

Im Unterschied zu Glukose wird Galaktose im menschlichen Körper insulinunabhängig in die Zellen transportiert. Der glykämische Index (GI) der Galaktose ist mit GI=20 im Vergleich zur Saccharose (GI=65) und Glukose (GI=100) sehr niedrig. Dagegen ist die Süßkraft von Galaktose mindestens halb so groß wie die von Glukose bzw. Saccharose: Bezogen auf Saccharose hat eine 10%-ige D-Galaktoselösung eine Süßkraft von 63 %.

Aufgrund seiner Eignung als kataboles Substrat, d.h. als Energielieferant, ist Galaktose vor allem für solche Patienten von Vorteil, die eine Insulinresistenz haben und bei denen es trotz eines hohen Blutzuckerspiegels zu einer Unterversorgung der Glukose abhängigen Nervenzellen insbesondere des Gehirns kommt - z.B. bei Patienten mit Morbus Alzheimer. Durch Verabreichung von Galaktose bzw. galaktosehaltigen Lebensmitteln kann solchen Patienten auf Insulin-unabhängige Weise Glukose indirekt zugeführt werden (die Galaktose wird Insulin-unabhängig über GluT-3-Transporter in die Nerven- und Hirnzellen geschleust und dort in Glukose umgebaut). Prinzipiell führt auch bei einem gesunden Menschen die Aufnahme von Galaktose wenigstens langfristig zu einer höheren muskulären und kognitiven Leistungsfähigkeit.

Galaktose bewirkt selbst nur eine relativ geringe Insulinausschüttung, und fördert so indirekt, nämlich über den relativ niedrigen Insulinspiegel, die Energieerzeugung mittels Fettverbrennung. Außerdem werden mit dem niedrigen Insulinausstoß Unterzuckerungszustände und dadurch hervorgerufene Heißhungerattacken vermieden. Deshalb eignet sich Galaktose sehr gut als Zuckerkomponente in der Ernährung von Menschen die ihr Gewicht kontrollieren oder Übergewicht reduzieren müssen.

Im Gegensatz zu Glukose und Fruktose kommt Galaktose in der Natur nur in geringen Mengen als Monosaccharid vor. Vom Menschen wird Galaktose herkömmlich fast ausschließlich in Form des Disaccharids Laktose konsumiert. Viele erwachsene Menschen haben jedoch einen Mangel an dem Enzym Laktase, das Laktose spaltet, und leiden deshalb an Laktoseunverträglichkeit. Aus diesem Grund verzichtet die Lebensmittelindustrie immer mehr auf die Verwendung von Laktose in Lebensmitteln. Damit wird jedoch auch der Anteil der Galaktose in der Ernährung des Menschen reduziert.

Obwohl Galaktose in Form des freien Monosaccharids für Menschen mit Laktasemangel verdaulich ist, und obwohl hochreine Galaktose im Stand der Technik zur Verfügung steht, wird das Monosaccharid Galaktose bisher nicht in Getränken eingesetzt. Ein Grund hierfür könnte darin liegen, dass Galaktose verdächtigt wird, in der Zelle die Radikalbildung zu fördern und eine Mitochondriendysfunktion auszulösen.

Andererseits haben Untersuchungen des Nobelpreisträgers Otto Heinrich Warburg gezeigt, dass der Vergärungsstoffwechsel von Krebszellen (in Krebsgewebeschnitten) mit Glukose als Nahrungssubstrat am effektivsten abläuft, nämlich mit einer Rate von 23,9 verbrauchte Einheiten pro Zeit, während er mit dem Substrat Fruktose um den Faktor 7 weniger effektiv (Rate 3,3) und mit Galaktose sogar um den Faktor 18 weniger effektiv (Rate 1,3) abläuft. Das bedeutet: Krebszellen, denen nur Galaktose als Nahrungssubstrat zur Verfügung steht, haben einen vergleichsweise drastisch verminderten Vergärungsstoffwechsel und infolgedessen eine entsprechend verminderte Wachstums- und Teilungsrate. Galaktose sollte deshalb in der Ernährung von Krebspatienten eine wesentliche Zuckerquelle sein.

Isomaltulose hat neben seinen niedrigen glykämischen Index außerdem die vorteilhaften Eigenschaften, dass sie eine ROS-neutralisierende Wirkung besitzt und dass sie von den Plaque-Bakterien im menschlichen Mund nicht gespalten werden, weshalb dort beim Verzehr von Isomaltulose keine zahnschädigenden Säuren entstehen.

Die Kombination von Isomaltulose mit Galaktose, die ebenfalls zahnfreundlicher ist als Saccharose, verleiht dem erfindungsgemäßen Getränk den weiteren Vorteil, dass es auch für Kinder und Jugendliche besonders geeignet ist, weil diese einerseits süße Getränke bevorzugen und andererseits ihre teilweise noch im Aufbau befindlichen Zähne gerade in dieser Entwicklungsphase vor Karies schützen sollten.

Tagatose ist ein natürlich vorkommendes Monosaccharid, das auch aus Galaktose mittels Isomerisierung hergestellt werden kann. Tagatose hat einen niedrigen glykämischen Index von GI = 3 und eine Süßkraft von etwa 97 % bezogen auf Saccharose, weist dabei aber einen deutlich geringeren Energieinhalt als Saccharose auf, weil es nur zu etwa 20% direkt vom menschlichen Körper verdaut wird. Die restlichen 80% der Tagatose werden von der Darmflora abgebaut, wobei unter anderem Butyrat gebildet wird, das eine hemmende Wirkung auf das Wachstums von Krebszellen hat, in Krebszellen Apoptose auslösen kann und den Zuckerstoffwechsel in Krebszellen steuert (Int J Cancer. 2010 Aug 16. Butyrate elicits a metabolic switch in human colon cancer cells by targeting the pyruvate dehydrogenase complex.) Fruktose und Tagatose sind im Stand der Technik dafür bekannt, dass sie die Bildung von reaktiven Sauerstoffspezies, sogenannten Sauerstoffradikalen (englisch: Reactive Oxygen Species = "ROS"), unterdrücken oder entstehende Sauerstoffradikale/ROS neutralisieren (Free Radic Biol Med. 1997;22(1-2):257-268: Fructose and tagatose protect against oxidative cell injury by iron chelation) und so einer möglichen ROS-Bildung durch Galaktose entgegenwirken.

Trehalose ist ein natürlicher zahnfreundlicher Zweifach-Zucker aus zwei Glucose-Molekülen (alpha 1-1 Bindung). Seine Süßkraft beträgt ca. 50% der Süßkraft von Saccharose. Der glykämische Index von Trehalose ist etwa so groß wie der von Isomaltulose (GI = 32). Genau wie Isomaltulose wird Trehalose erst im menschlichen Dünndarm abgebaut und bewirkt einen niedrigen Insulinausstoß. Trehalose ist ein nichtreduzierender Zucker und reagiert infolgedessen nicht mit freien Aminogruppen von Aminosäuren, wodurch die Trehalose enthaltenden Lebensmittel stabiler sind. Da Trehalose aufgrund seiner nicht-reduzierenden Eigenschaften nicht mit anderen Lebensmittelbestandteilen reagiert, werden sowohl diese anderen Lebensmittel-Inhaitsstoffe als auch Trehalose selbst nicht chemisch modifiziert und stehen damit in unveränderter, biologisch wirksamer Form zur Verfügung. Da beim Abbau von Trehalose nur Glukose freigesetzt wird, kann Trehalose auch bei Fruktose-Unverträglichkeit als Süßungsmittel eingesetzt werden. Trehalose verleiht Lebensmitteln ein angenehmes Mundgefühl. Zudem unterdrückt Trehalose Geschmacksnoten, die von vielen Menschen als eher unangenehm empfunden werden wie z.B. leicht bitteren Geschmack. Damit geht der Vorteil einher, dass Trehalose-haltigen Erfrischungsgetränken gesundheitsfördernde, aber wenig angenehm schmeckende Inhaltsstoffe wie sekundäre Pflanzenstoffe und Tocotrienole dennoch in höherer Konzentration zugegeben werden können.

Ribose kommt in allen pflanzlichen und tierischen Zellen vor. Im tierischen Körper wird D-Ribose unter anderem zur Bildung von Adenosintriphosphat (ATP), dem wichtigsten Energielieferant des Stoffwechsels genutzt. In Sportlerkreisen wird D-Ribose als Nahrungsergänzung eingesetzt, um das bei der Muskeltätigkeit verbrauchte ATP schneller nachzuproduzieren und damit die Regenerationsphase zwischen Trainingseinheiten zu verkürzen. D-Ribose wird wie Galaktose völlig anders verstoffwechselt als Saccharose und ihre Einnahme bewirkt keinen Anstieg, sondern vielmehr eine Absinkung des Blutzuckerspiegel. Ribose besitzt damit sogar einen negativen glykämischen Index, weil nach dem Konsum von Ribose der Blutzuckerspiegel gesenkt wird. Dies kann ausgenutzt werden, um den glykämischen Index des Erfrischungsgetränkes zu senken. Die Süßkraft von Ribose beträgt ca. 30% der Süßkraft von Saccharose.

Die Gluconsäure in dem Getränk bewirkt dessen frischen, leicht säuerlichen Geschmack, d.h. sie wirkt ähnlich wie Phosphorsäure, ohne aber die für Phosphorsäure bekannten Nachteile hervorzurufen. Gluconsäure, eine organische, milde Säure, ist ein natürliches Produkt des Kohlenhydrat-Stoffwechsels im menschlichen Körper und kann biotechnologisch mit Hilfe von Mikroorganismen oder Enzymen aus Glukose (Traubenzucker) hergestellt werden. Im Gegensatz zu Phosphorsäure kann der menschliche Stoffwechsel aus Gluconsäure langsam und gleichmäßig Energie für anabole und katabole Reaktionen bereitstellen. Phosphorsäure dagegen liefert keine Energie, schädigt den Körper aufgrund der starken Säureeigenschaften und belastet zudem den Mineralienhaushalt des Körpers, da die über Phosphorsäure aufgenommene Menge an Phosphat aus dem Körper wieder eliminiert werden muss. Gluconsäure dagegen wird im menschlichen Körper komplett zu Wasser und Kohlendioxid abgebaut und belastet den Mineralhaushalt des Köpers nicht, da sie keine Mineralien enthält. Im Stand der Ernährungswissenschaften wird Gluconsäure als gesundheitlich unbedenklich eingestuft. Die Kombination von Galaktose mit Isomaltulose und/oder Tagatose und/oder Trehalose und/oder Ribose als Zuckerkomponente verleiht dem neuen Getränk die vorteilhafte Eigenschaft, dass es süß schmeckt, nach dem Verzehr im menschlichen Stoffwechsel aber nur einen geringeren Insulinausstoß verursacht. Der geringe Insulinausstoß provoziert eine erhöhte Fettverbrennung und eine erhöhte Aktivität der Mitochondrien, was insbesondere für Menschen mit Übergewicht sehr von Vorteil ist. Die erfindungsgemäße Zuckerkombination hat außerdem die vorteilhafte Eigenschaft, dass sie eine hemmende Wirkung auf das Wachstum von Krebsgeschwüren, insbesondere von TKTL1-positiven Krebsgeschwüren entfaltet, ohne dabei zellschädigende Effekte in gesunden Zellen auszulösen, und dass sie über die provozierte Aktivität der Mitochondrien die Wirkung von Strahlen- und Chemotherapien bei der Krebsbekämpfung erhöht.

Das Risiko zur Entwicklung von Diabetes Typ-2, Metabolischem Syndrom und auch Bluthochdruck kann durch eine Flüssigkeitszufuhr in Form des erfindungsgemäßen Getränks gesenkt werden, insbesondere wenn die bisher konsumierten geschmacklich vergleichbaren herkömmlichen Getränke zur Deckung des täglichen Flüssigkeitsbedarfs vollständig oder nahezu vollständig damit ersetzt werden. Gleichzeitig eignet sich das erfindungsgemäße Getränk als Getränke-Komponente in Ernährungstherapien zur Bekämpfung von Krebsgeschwüren, insbesondere von TKTL1-positiven Tumoren und/oder Metastasen, wobei diese Ernährungstherapien auf dem Prinzip beruhen, dass solche Kohlenhydrate, die im Stoffwechsel leicht und schnell in Glukose überführt werden können, drastisch reduziert sind.

In einer bevorzugten Ausführungsform weist das erfindungsgemäße Getränk zusätzlich einen Gehalt an Fruchtsäften oder Fruchtsaftextrakten auf.

Um trotz der zugesetzten Fruchtsäfte oder Fruchtsaftextrakten die maximale Obergrenze für den Zuckergehalt des Getränks einzuhalten, kann ein Teil der in diesen Fruchtsäften oder Fruchtsaftextrakten enthaltenen Zucker durch Fermentation in Gluconsäure umgewandelt werden.

In einer bevorzugten Ausführungsvariante ist zusätzlich ein Gehalt an Stevia vorgesehen. Stevia (Synonyme: Süßkraut, Süßblatt und Honigkraut) ist die allgemein übliche Kurzbezeichnung für Stevia rebaudiana bertoni (Gattung: Stevien, Familie: Korbblütler). Die Blätter dieser Pflanze enthalten das Diterpenglykosid Steviosid, das die bis zu 300-fache Süßkraft von Zucker hat, die Zähne vor Karies schützt und den Insulinsspiegel nicht beeinflusst. Zur Süßung werden die getrockneten Blätter oder die industriell gewonnenen Süß-Extrakte oder Derivate davon verwendet. Diese Variante des Getränks ist aufgrund ihres ausgeprägten Süßgeschmacks und des Kariesschutzes besonders für Kinder geeignet und vorgesehen.

In einer weiteren Ausführungsform des erfindungsgemäßen Getränks, die insbesondere für Cola-Liebhaber vorgesehen ist, weist das Getränk einen Gehalt an Zuckerkulör und einen Gehalt an natürlichen Aromastoffen auf und hat einen Cola-artigen Geschmack.

Bei den Aromastoffen handelt es sich vorzugsweise um Extrakte aus Ackerminze und/oder Cocablatt und/oder Galgant und/oder Ingwer und/oder Kardamom und/oder Kolanuss und/oder Kakao und/oder Limette und/oder Muskatblüte und/oder Nelke und/oder Orange und/oder Pinie und/oder Senfsamen und/oder Süßholz und/oder Vanille und/oder Zitrone und/oder Zimt.

In einer weiteren bevorzugten Ausführungsform weist das Getränk zusätzlich einen Gehalt an Vitamin B1 in Form des Thiaminderivats Benfotiamin auf. Benfotiamin unterdrückt die ROS-Bildung in Zellen und wirkt damit einer möglichen ROS-Bildung durch Galaktose entgegen (Diabetes Metab Res Rev. 2008 Jul-Aug;24(5):371-7 - Benfotiamine exhibits direct antioxidative capacity and prevents induction of DNA damage in vitro). Darüber hinaus hat Benfotiamin eine deutlich bessere biologische Wirksamkeit als Thiamin und bewirkt insbesondere, dass der Ab- und Umbau der in dem Getränk enthaltenen Zuckern beschleunigt wird und damit die negativen Effekte von Zuckern (advanced glucose endproducts = AGE), insbesondere die chronischen Diabetesschäden, reduziert oder vermieden werden.

Das erfindungsgemäße Getränk kann zusätzlich einen Gehalt an Vitamin E in Form einer Tocotrienol-Tocopherol-Mischung aus natürlichen Quellen aufweisen.

Vitamin E in Form des alpha-Tocopherols ist im Stand der Technik dafür bekannt, dass es sehr effektiv die Bildung von reaktiven Sauerstoffspezies, sogenannten Sauerstoffradikalen (englisch: Reactive Oxygen Species = "ROS"), unterdrückt oder entstehende Sauerstoffradikale/ROS neutralisiert (Diabetes. 1996 Sep;45(9):1233-7. Abnormalities of retinal metabolism in diabetes or experimental galactosemia. III. Effects of antioxidants; Free Radic Res. 2000 Jan;32(1):67-74. Diabetes-induced metabolic abnormalities in myocardium: effect of antioxidant therapy.) Eine etwaige zellschädigende Wirkung des Monosaccharids Galaktose wird durch die kombinierte gleichzeitige Aufnahme von Vitamin E in Form der natürlichen Tocotrienol-Tocopherol-Mischung verhindert oder kompensiert.

Die Tocotrienol-Tocopherol-Mischung hat außerdem den Vorteil, dass ihre Tocotrienole, insbesondere das Gamma-Tocotrienol und das Delta-Tocotrienol jüngeren Forschungsergebnissen zufolge bei verschiedenen Krebsarten als Inhibitor der Zellproliferation und als Auslöser der Apoptose wirken und damit eine direkte Anti-Krebswirkung aufweisen (z.B. Pharmacology. 2010;85(4):248-58; BMC Cancer. 2010 Mar 8;10:84; Breast Cancer Res Treat. 2010 Feb 16).

Als Quelle für die Tocotrienol-Tocopherol-Mischung kommen insbesondere Palmöl und Reiskleie oder Tocopherol-Tocotrienol-Extrakte aus Palmöl und Reiskleie in Betracht.

In einer weiteren Ausführungsvariante weist das Getränk zusätzlich einen Gehalt an Omega-3-Fettsäuren und/oder mittelkettigen Triglyceriden ("MCT") auf, womit seine gesundheitsfördernde Wirkung noch gesteigert wird.

Die Omega-3-Fettsäuren liegen vorzugsweise in Form von Ölsaaten/Ölsamen vor. Unter "Ölsaaten"/"Ölsamen" werden Pflanzensamen bezeichnet, die zur Gewinnung von Pflanzenöl genutzt werden können. Zu den Ölsaaten/Ölsamen zählen u.a. Soja, Raps, Hanf, Flachs, Walnuss. Die Samen können im Ganzen oder als Ölschrot eingesetzt werden. "Ölschrot" bezeichnet die bei der Verarbeitung von Ölsaaten und Ölfrüchten neben dem Öl gewonnenen Koppelprodukte. Je nach Verarbeitungsverfahren wird Ölschrot auch Presskuchen (bei Heiß- oder Kaltpressung der Ölsaaten) oder Extraktionsschrot (bei Ölextraktion durch Lösungsmittel) genannt. Ölsaaten zeichnen sich durch einen hohen Gehalt an essentiellen ungesättigten Fettsäuren und essentiellen Aminosäuren aus und enthalten darüber hinaus auch Mineralien wie Magnesium, Selen oder Zink. Für einige Ölsaaten ist eine direkte Anti-Krebs-Wirkung beschrieben worden, und deshalb werden solche Ölsaaten oder auch die entsprechenden Öle bevorzugt eingesetzt.

MCT fördern die Bereitstellung von Ketonkörpern und von freien mittelkettigen Fettsäuren, die beide eine wichtige Rolle im Energiestoffwechsel haben, insbesondere als alternative Energiequelle zu Blutzucker einerseits bei Krebspatienten mit erhöhter Aktivität des TKTL1-Gens im Energiestoffwechsel des gesunden Gewebes und andererseits bei Menschen mit Insulinresistenz im Energiestoffwechsel vor allem der Nervenzellen des Gehirns (aufgrund ihrer Passagemöglichkeit der Blut-Him-Schranke). Mittelkettige Triglyceride sind erfindungsgemäß Triglyceride mit einer Kettenlänge von bevorzugt C8 und/oder C10.

Das Getränk kann ferner einen zusätzlichen Gehalt an sekundären Pflanzenstoffen ("SPS") aufweisen und vorzugsweise eines oder mehrere Mitglieder der folgenden Gruppe enthalten: Glucosinolate, Carotinoide, Lektine, Flavonoide, Phytosterine, Polyphenolen, insbesondere Curcumin, Ellagsäure, Quercetin, Resveratrol, Delphinidin, Diallylsulfid, Epigallocatechin-3-gallat, Genistein, Indol-3-carbinol, Isoterpene, Limonin, Lycopin, OPC, Salvestrole, Sulforaphan.

Sekundäre Pflanzenstoffe besitzen neueren Erkenntnissen zufolge eine bedeutende Wirksamkeit, insbesondere bei chronischen Entzündungen und Mitochondrienschädigungen sowie bei onkologischen Erkrankungen. Um diese Wirkung zu erzielen, sollten die sekundären Pflanzenstoffe nicht isoliert oder naturidentisch verabreicht oder einem Nahrungsmittel zugesetzt werden, sondern sie sollten in Form ihrer natürlichen Quellen, vorzugsweise in Form von Extrakten aus diesen natürlichen Quellen aufgenommen bzw. dem Nahrungsmittel, das heißt hier dem erfindungsgemäßen Getränk zugesetzt werden.

Das Getränk kann außerdem einen zusätzlichen Gehalt an CO₂-extrahierten Aromen, insbesondere aus Himbeere und/oder Kiwi und/oder Hagebutten und/oder Ingwer und/oder Holunder und/oder Litschi aufweisen. CO₂-Extrakte weisen im Gegensatz zu den mit klassischen Ölmühlen erzeugten Ölen keine Metallpartikel aus dem mechanischen Abrieb der Ölmühlen auf, stattdessen aber zum Teil wesentlich höhere Konzentrationen an sekundären Pflanzenstoffen.

Das Getränk kann ferner einen zusätzlichen Gehalt an Vitamin D aufweisen, um einem Vitamin-D-Mangel vorzubeugen oder entgegen zu wirken, denn Vitamin D-Mangel fördert Insulinresistenz und Metabolisches Syndrom, und um die bekannte starke Anti-Krebswirkung von Vitamin D zu nutzen.

Um die mitochondriale Energieproduktion und Fettverbrennung zu erhöhen und gleichzeitig die unerwünschte Vergärung in Krebszellen zu hemmen, kann das Getränk zusätzlich einen Gehalt an Carnitin und/oder Kreatin aufweisen.

In einer Ausführungsform, in der das Getränk ein besonders spritziges und erfrischendes Gefühl beim Trinken vermittelt, weist es zusätzlich einen Gehalt an Kohlensäure auf.

Um das Bedürfnis der Verbraucher nach einem anregenden Getränk zu erfüllen, ist eine Ausführungsvariante vorgesehen, in der das Getränk einen Gehalt an Koffein und/oder an einem koffeinhaltigen Extrakt aufweist. Bevorzugt wird Koffein in Form von Extrakten aus natürlichen Quellen wie Guarana dem Getränk zugesetzt, weil so das Koffein während des Verdauungsprozesses im Körper und somit über einen längeren Zeitraum freigesetzt wird und nicht als freies, direkt verfügbare Koffein vorliegt.

Die Zusammensetzung des erfindungsgemäßen Getränks basiert vorzugsweise ganz auf natürlichen Inhaltsstoffen, was neben den oben erwähnten Eigenschaften auch zur besseren Verträglichkeit und Akzeptanz in den angesprochenen Verkehrskreisen führt.

Im Folgenden wird das erfindungsgemäße Getränk anhand von Rezeptur-Beispielen näher erläutert.

Die in den Rezepturen angegeben Mengenangaben gelten pro 100 Milliliter Getränk.

### Beispiel 1: Erfrischungsgetränk - Mineralarm

Zutaten:
0,4 % Gluconsäure
1 g Galaktose
0,4 g Isomaltulose
0,9 g Tagatose
1 % Aroniasaft - partiell zu Gluconsäure fermentiert

### Beispiel 2: Fruchtsafterfrischuagsgetränk Schwarze Johannisbeere

Zutaten:
0,3 % Gluconsäure
0,9 g Galaktose
0,3 g Isomaltulose
1 g Tagatose
0,7 % Aroniasaft - partiell zu Gluconsäure fermentiert
0,6 % Schwarze-Johannisbeersaft - partiell zu Gluconsäure fermentiert

### Beispiel 3: Fruchtsafterfrischungsgetränk Multifrucht

Zutaten:
0,6 % Gluconsäure
0,8 g Galaktose
0,2 g Isomaltulose
1,0 g Tagatose
0,3 % Schwarze-Johannisbeersaft - partiell zu Gluconsäure fermentiert
0,3 % Kirschsaft - partiell zu Gluconsäure fermentiert
0,3 % Birnensaft - partiell zu Gluconsäure fermentiert
0,05 % CO2-Ingwerextrakt (Zitronengeschmack)
Kohlensäure

### Beispiel 4: Fruchtsafterfrischungsgetränk Ingwer-Orange

Zutaten:
0,3 % Gluconsäure
0,5 g Galaktose
0,2 g Isomaltulose
1,5 g Tagatose
1 % Orangensaft - partiell zu Gluconsäure fermentiert
0,05 % CO2-Ingwerextrakt (Zitronengeschmack)
1 % MCT
Kohlensäure

### Beispiel 5: Fruchtsafterfrischungsgetränk Himbeere-Johannisbeere I

Zutaten:
1,3 % Gluconsäure
0,7 g Galaktose
0,3 g Isomaltulose
2,3 % Aroniasaft - partiell zu Gluconsäure fermentiert
2,3 % Schwarze Johannisbeere - partiell zu Gluconsäure fermentiert
0,05 % CO2-Himbeerkemextrakt (omega-3-fettsäurereich)
1,2 % MCT
Kohlensäure

### Beispiel 6: Fruchtsafterfrischungsgetränk Himbeere-Johannisbeere II

Zutaten:
1,3 % Gluconsäure
0,7 g Galaktose
0,3 g Isomaltulose
1,2 % Tagatose
0,3 % Aroniasaft - partiell zu Gluconsäure fermentiert
0,3 % Schwarze Johannisbeere - partiell zu Gluconsäure fermentiert
0,05 % CO2-Himbeerkernextrakt (omega-3-fettsäurereich)
1,2 % MCT
0,5% natürliche Aromen aus Ackerminze und Galgant und Ingwer und Kardamom und Kolanuss und Limette und Muskatblüte und Nelke und Orange und Pinie und Senfsamen und Süßholz und Vanille und Zitrone und Zimt.
Kohlensäure

### Beispiel 7: Cota-Getränk I

Zutaten:
2,5 % Gluconsäure
0,6 g Galaktose
0,3 g Isomaltulose
1,3 g Tagatose
3 % Zuckerkulör
0,5% natürliche Aromen aus Ackerminze und Galgant und Ingwer und Kardamom und Kolanuss und Limette und Muskatblüte und Nelke und Orange und Pinie und Senfsamen und Süßholz und Vanille und Zitrone und Zimt
Kohlensäure

### Beispiel 8: Cola-Getränk II

Zutaten:
2,5 % Gluconsäure
0,8 g Galaktose
0,3 g Isomaltulose
1,3 g Tagatose
3 % Zuckerkulör
0,5% natürliche Aromen aus Ackerminze und Galgant und Ingwer und Kardamom und Kolanuss und Limette und Muskatblüte und Nelke und Orange und Pinie und Senfsamen und Süßholz und Vanille und Zitrone und Zimt
0,2% Stevia
Kohlensäure

### Beispiel 9: Cola-Getränk III

Zutaten:
2,5 % Gluconsäure
0,7 g Galaktose
0,4 g Isomaltulose
1,3 g Tagatose
3 % Zuckerkulör
0,5% natürliche Aromen aus Ackerminze und Galgant und Ingwer und Kardamom und Kolanuss und Limette und Muskatblüte und Nelke und Orange und Pinie und Senfsamen und Süßholz und Vanille und Zitrone und Zimt
0,2% Stevia
5 mg Benfotiamin
Kohlensäure

### Beispiel 10: Cola-Getränk IV

Zutaten:
2,5 % Gluconsäure
0,7 g Galaktose
0,3 g Isomaltulose
1,3 g Tagatose
3 % Zuckerkulör
0,5% natürliche Aromen aus Ackerminze und Galgant und Ingwer und Kardamom und Kolanuss und Limette und Muskatblüte und Nelke und Orange und Pinie und Senfsamen und Süßholz und Vanille und Zitrone und Zimt
0,2% Stevia
5 mg Benfotiamin
1% MCT
Kohlensäure

### Beispiel 11: Cola-Getränk V

Zutaten:
2,5 % Gluconsäure
0,8 g Galaktose
0,4 g Isomaltulose
1,1 g Tagatose
1,8 % Zuckerkulör
0,5% natürliche Aromen aus Ackerminze und Galgant und Ingwer und Kardamom und Kolanuss und Limette und Muskatblüte und Nelke und Orange und Pinie und Senfsamen und Süßholz und Vanille und Zitrone und Zimt 0,2% Stevia
15 mg Benfotiamin
25 mg Carnitin
65 mg Kreatin
2%MCT
Kohlensäure

### Beispiel 12: Cola-Getränk VI

Zutaten:
0,5 % Gluconsäure
0,3 g Galaktose
0,6 Trehalose
0,4 g Isomaltulose
0,6 g Tagatose
0,1 Ribose
1,0 g Erythritol
0,8 % Zuckerkulör
0,5 % natürliche Aromen aus Ackerminze und Galgant und Ingwer und Kardamom und Kolanuss und Limette und Muskatblüte und Nelke und Orange und Pinie und Senfsamen und Süßholz und Vanille und Zitrone und Zimt
0,2% Stevia
15 mg Benfotiamin
25 mg Carnitin
65 mg Kreatin
2% MCT
Kohlensäure

### Beispiel 13: Cola-Getränk VII

Zutaten:
0,5 % Gluconsäure
0,5 g Galaktose
0,6 Trehalose
0,4 g Isomaltulose
0,6 g Tagatose
0,1 Ribose
1,0 g Erythritol
0,7 % Zuckerkulör
0,5% natürliche Aromen aus Ackerminze und Galgant und Ingwer und Kardamom und Kolanuss und Limette und Muskatblüte und Nelke und Orange und Pinie und Senfsamen und Süßholz und Vanille und Zitrone und Zimt
0,2% Stevia
15 mg Benfotiamin
25 mg Carnitin
65 mg Kreatin
2% MCT
Kohlensäure

### Beispiel 14: Erfrischungsgetränk Goji-Johannisbeere-Orangenaroma

Zutaten:
0,5 % Gluconsäure
0,5 g Galaktose
0,7 Trehalose
0,4 g Isomaltulose
0,6 g Tagatose
0,1 Ribose
1,0 g Erythritol
0,2% Stevia
0,1 % Goji-Saft
0,3 % Schwarze Johannisbeere - partiell zu Gluconsäure fermentiert
0,01 % natürliches Orangenaroma
15 mg Benfotiamin
25 mg Carnitin
65 mg Kreatin
2% MCT
Kohlensäure

### Beispiel 15: Erfrischungsgetränk - Mineralarm

Zutaten:
0,4 % Gluconsäure
0,5 g Galaktose
0,8 g Trehalose
0,4 g Isomaltulose
0,1 g Ribose
0,4 g Tagatose
0,6 % Aroniasaft - partiell zu Gluconsäure fermentiert
0,3% Stevia

## Patentansprüche

1. Phosphorsäurefreies, mineralarmes, zuckerarmes, süß schmeckendes Erfrischungsgetränk, **dadurch gekennzeichnet,**
- **dass** es einen Gehalt an Gluconsäure von mindestens 0,3 % Gew./Vol. aufweist,
- **dass** ein etwaiger Gehalt an Mineralien weniger als 250 mg pro Liter beträgt,
- **dass** es einen Gehalt an Zuckern von maximal 2,5 % Gew./Vol. aufweist,
- **dass** der Gehalt an Zuckern einen Anteil Galaktose umfasst, der mindestens 10 % Gew./Gew. bezogen auf den Gehalt an Zuckern beträgt, dass er zusätzlich einen Gehalt an Isomaltulose und/oder Tagatose und/oder Trehalose und/oder Ribose umfasst,
- und **dass** das Getränk einen glykämischen Index von maximal 35 (GI<35) aufweist.

2. Getränk nach Anspruch 1, **dadurch gekennzeichnet, dass** der Gehalt an Mineralien weniger als 150 mg pro Liter, vorzugsweise weniger als 100 mg pro Liter beträgt.

3. Getränk nach Anspruch 1 oder 2, **dadurch gekennzeichnet, dass** es einen Gehalt an Fruchtsäften oder Fruchtsaftextrakten aufweist.

4. Getränk nach einem der Ansprüche 1 bis 3, **dadurch gekennzeichnet, dass** es zusätzlich einen Gehalt an Stevia aufweist.

5. Getränk nach einem der Ansprüche 1 bis 4, **dadurch gekennzeichnet, dass** es einen Gehalt an Zuckerkulör und natürlichen Aromastoffen umfasst und einen Cola-artigen Geschmack aufweist.

6. Getränk nach Anspruch 5, **dadurch gekennzeichnet, dass** die Aromastoffe Ackerminze und/oder Cocablatt und/oder Galgant und/oder Ingwer und/oder und/oder Kardamom und/oder Kolanuss und/oder Kakao und/oder Limette und/oder Muskatblüte und/oder Nelke und/oder und/oder Orange und/oder Pinie und/oder Senfsamen und/oder Süßholz und/oder Vanille und/oder Zitrone und/oder Zimt oder Extrakte davon sind.

7. Getränk nach einem der Ansprüche 1 bis 6, **dadurch gekennzeichnet, dass** es zusätzlich einen Gehalt an Benfotiamin aufweist.

8. Getränk nach einem der Ansprüche 1 bis 7, **dadurch gekennzeichnet, dass** es zusätzlich einen Gehalt an einer Tocotrienol-Tocopherol-Mischung aus natürlichen Quellen aufweist.

9. Getränk nach einem der Ansprüche 1 bis 8, **dadurch gekennzeichnet, dass** es zusätzlich einen Gehalt an Omega-3-Fettsäuren und/oder mittelkettigen Triglyceriden ("MCT") aufweist.

10. Getränk nach einem der Ansprüche 1 bis 9, **dadurch gekennzeichnet, dass** es zusätzlich einen Gehalt an sekundären Pflanzenstoffen aufweist, die ausgewählt sind aus der Gruppe umfassend: Glucosinolate, Carotinoide, Lektine, Flavonoide, Phytosterine, Polyphenolen, insbesondere Curcumin, Ellagsäure, Quercetin, Resveratrol, Delphinidin, Diallylsulfid, Epigallocatechin-3-gallat, Genistein, Indol-3-carbinol, Isoterpene, Limonin, Lycopin, OPC, Salvestrole, Sulforaphan und Ubichinon.

11. Getränk nach einem der Ansprüche 1 bis 10, **dadurch gekennzeichnet, dass** es einen Gehalt an CO₂-exctrahierten Aromen aufweist, vorzugsweise Aromen aus Himbeere und/oder Kiwi und/oder Hagebutte und/oder Ingwer und/oder Holunder und/oder Litschi.

12. Getränk nach einem der Ansprüche 1 bis 11, **dadurch gekennzeichnet, dass** es zusätzlich einen Gehalt an Vitamin D aufweist.

13. Getränk nach einem der Ansprüche 1 bis 12, **dadurch gekennzeichnet, dass** es zusätzlich einen Gehalt an Carnitin und/oder Kreatin aufweist.

14. Getränk nach einem der Ansprüche 1 bis 13, **dadurch gekennzeichnet, dass** es Kohlensäure enthält.

15. Getränk nach einem der Ansprüche 1 bis 14, **dadurch gekennzeichnet, dass** es zusätzlich einen Gehalt an Koffein oder wenigstens eines koffeinhaltigen Extraktes (z.B. Guarana) enthält.

16. Getränk nach einem der Ansprüche 1 bis 15, **dadurch gekennzeichnet, dass** es frei von künstlichen Süßstoffen, künstlichen Farbstoffen und künstlichen Aromen ist.

17. Getränk nach einem der Ansprüche 1 bis 16 zur Anwendung in der präventiven oder therapeutischen Behandlung von Krankheiten aus der Gruppe umfassend: Zöliakie, Diabetes mellitus Typ-2, neurodegenerative Krankheiten, insbesondere Alzheimer, Tumorerkrankungen, sowie Erkrankungen, die zu einer Kachexie (Auszehrung) führen, die mit entzündlichen Prozessen einhergehen oder davon verursacht werden, insbesondere Rheuma, rheumatoide Arthritis, entzündliche Darmerkrankungen wie Ulzerative Colitis, Morbus Crohn, Darmerkrankungen wie das Leaky-Gut-Syndrom (durchlässiger Darm).

## Claims

1. A sweet-tasting refreshing beverage that is free from phosphoric acid, is low in minerals and is low in sugar, **characterized in that**,
- it has a content of gluconic acid that is at least 0.3 wt/vol %,
- **in that** any content of minerals is less than 250 mg/liter,
- **in that** it has a content of sugars of at most 2.5 wt/vol %,
- **in that** the content of sugars comprises a fraction of galactose that is at least 10 weight-weight % based on the content of sugars, **in that** it additionally comprises a content of Isomaltulose and/or tagatose and/or trehalose and/or ribose,
- and **in that** the beverage has a glycemic index of at most 35 (GI < 35).

2. The beverage as claimed in claim 1, **characterized in that** the content of minerals is less than 150 mg per liter, preferably less than 100 mg per liter.

3. The beverage as claimed in claim 1 or 2, **characterized in that** it has a content of fruit juices or fruit juice extracts.

4. The beverage as claimed in one of claims 1 to 3, **characterized in that** it additionally has a content af Stevia.

5. The beverage as claimed in one of claims 1 to 4, **characterized in that** it comprises a content of caramel coloring and natural flavorings and has a cola-like taste.

6. The beverage as claimed in claim 5, **characterized in that** the flavorings are Mentha arvensis and/or coca leaf and/or galangal and/or ginger and/or cardamom and/or kola nut and/or cocoa and/or lime and/or mace and/or clove and/or orange and/or pine and/or mustard seed and/or licorice and/or vunilla and/or lemon and/or cinnamon or extracts thereof.

7. The beverage as claimed in one of claims 1 to 6, **characterized in that** it additionally has a content of benfotiamine.

8. The leverage as claimed in one of claims 1 to 7, **characterized in that** it additionally has a content of a tocotrienol-tocopherol mixture from natural sources.

9. The beverage as claimed in one of claims 1 to 8, **characterized in that** it additionally has a content of omega-3 fatty acids and/or medium-chain triglycerides ("MCTs").

10. The beverage as claimed in one of claims 1 to 9, **characterized in that characterized in that** it additionally has a content of secondary plant substances, which are selected from the group comprising: glucosinolates, carotenoids, lectins, flavonoids, phytosterols, polyphenols, in particular curcumin, ellagic acid, quercetin, resveratrol, delphinidin, diallyl sulfide, epigallocatechin-3-gallate, genistein, indol-3-carbinol, isoterpene, limonene, lycopene, OPC, salvestrol, sulforaphane and ubiquinone.

11. The beverage as claimed in one of claims 1 to 10, **characterized in that** it has a content of CO₂-extracted flavors, preferably flavors from raspberry and/or kiwi and/or rose hip and/or ginger and/or elder and/or lychee.

12. The beverage as claimed in one of claims 1 to 11, **characterized in that** it additionally has a content of vitamin D.

13. The beverage as claimed in one of claims 1 to 12, **characterized in that** it additionally has a content of carnitine and/or creatine.

14. The beverage as claimed in one of claims 1 to 13, **characterized in that** it contains carbon dioxide.

15. The beverage as claimed in one of claims 1 to 14, **characterized in that** it additionally contains a content of caffeine or of at least one extract containing caffeine (for example guarana).

16. The beverage as claimed in one of claims 1 to 15, **characterized in that** it is free from artificial sweeteners, artificial colorants and artificial flavorings.

17. Beverage as claimed in one of claims 1 to 16 for use in the preventative or therapeutic treatment of illnesses from the group comprising: celiac disease, type 2 diabetes mellitus, neurodegenerative diseases, in particular Alzheimer's disease, tumors, and illnesses that lead to cachexia (wasting syndrome), which accompany inflammatory processes or are caused thereby, in particular rheumatism, rheumatoid arthritis, inflammatory intestinal diseases such as ulecrative colitis and crohn's disease, and intestinal diseases such as leaky-gut syndrome (bowel hyperpermeability).

## Revendications

1. Boisson rafraîchissante exempte d'acide phosphorique, pauvre en minéraux, pauvre en sucres, ayant un goût sucré, **caractérisée en ce que**,
- elle comprend une teneur en acide gluconique d'au moins 0,3 % poids/volume,
- **en ce que** la teneur éventuelle en minéraux est inférieure à 250 mg par litre,
- **en ce qu'**elle comprend une teneur en sucres d'au maximum 2,5 % poids/volume,
- **en ce que** la teneur en sucres comprend une fraction de galactose qui s'élève à au moins 10% poids/poids rapporté à la teneur en sucres, **en ce que** la teneur en sucres comprend en outre une fraction d'isomaltulose et/ou de tagatose et/ou de tréhalose et/ou de ribose,
- et **en ce que** la boisson présente un index glycémique d'au plus 35 (GI < 35).

2. Boisson selon la revendication 1, **caractérisée en ce que** la teneur en minéraux est inférieure à 150 mg par litre, de préférence inférieure à 100 mg par litre.

3. Boisson selon la revendication 1 ou 2, **caractérisée en ce qu**'elle contient du jus de fruits ou en extraits de jus de fruits.

4. Boisson selon l'une des revendications 1 à 3, **caractérisée en ce qu**'elle contient en outre de la stevia.

5. Boisson selon l'une des revendications 1 à 4, **caractérisée en ce qu**'elle contient du colorant caramel et des arômes naturels et présente un goût de type cola.

6. Boisson selon la revendication 5, **caractérisée en ce que** les arômes naturels sont de la menthe des champs et/ou de la feuille de coca et/ou du galanga et/ou du gingembre et/ou de la cardamone et/ou de la noix de cola et/ou du cacao et/ou de la limette et/ou de la fleur de muscade et/ou du clou de girofle et/ou de l'orange et/ou du pin et/ou des graines de moutarde et/ou de la réglisse et/ou de la vanille et/ou du citron et/ou de la cannelle, ou des extraits de ces produits.

7. Boisson selon l'une des revendications 1 à 6, **caractérisée en ce qu**'elle contient en outre de la benfotiamine.

8. Boisson selon l'une des revendications 1 à 7, **caractérisée en ce qu**'elle contient en outre un mélange tocotriénol-tocophérol d'origine naturelle.

9. Boisson selon l'une des revendications 1 à 8, **caractérisée en ce qu**'elle contient en outre des acides gras oméga-3 et/ou des triglycérides à chaines moyennes («TCM»).

10. Boisson selon l'une des revendications 1 à 9 **caractérisée en ce qu**'elle contient en outre des métabolites végétaux secondaires qui sont choisis dans le groupe composé par : les glucosinolates, les caroténoïdes, les lectines, les flavonoïdes, les phytostérines, les polyphénols, notamment la curcumine, l'acide ellagique, la quercétine, le resvératrol, la delphinidine, le disulfure d'allyle, l'épigallocatéchine-3-gallate, la génistéine, l'indole-3-carbinol, les isoterpènes, la limonine, le lycopène, les OPC, les salvestrols, le sulforaphane et l'ubiquinone.

11. Boisson selon l'une des revendications 1 à 10, **caractérisée en ce qu**'elle contient des arômes extraits au CO₂, de préférence des arômes issus de de la framboise et/ou du kiwi et/ou du cynorrhodon et/ou du gingembre et/ou du sureau et/ou du litchi.

12. Boisson selon l'une des revendications 1 à 11, **caractérisée en ce qu**'elle contient en outre de la vitamine D.

13. Boisson selon l'une des revendications 1 à 12, **caractérisée en ce qu**'elle contient en outre de la carnitine et/ou de la créatine.

14. Boisson selon l'une des revendications 1 à 13, **caractérisée en ce qu**'elle contient du gaz carbonique.

15. Boisson selon l'une des revendications 1 à 14, **caractérisée en ce qu**'elle contient en outre de la caféine ou au moins un extrait contenant de la caféine (par exemple du guarana).

16. Boissons selon l'une des revendications 1 à 15, **caractérisée en ce qu**'elle est exempte d'édulcorants de synthèse, de colorants de synthèse et d'arômes de synthèse.

17. Boisson selon l'une des revendications 1 à 16 destinée à être utilisée pour le traitement préventif ou thérapeutique de maladies choisies dans le groupe composé par :
la maladie coeliaque, le diabète de type 2, les maladies neurodégénératives, notamment l'Alzheimer, les maladies tumorales, ainsi que les maladies qui conduisent à une cachéxie (amaigrissement), qui accompagnent des processus inflammatoires ou qui sont provoquées par ceux-ci, notamment le rhumatisme, l'arthrite rhumatoïde, les maladies inflammatoires de l'intestin telles que la colite ulcéreuse, la maladie de Crohn, les maladies intestinales telle le syndrome leaky-gut (hyperperméabilité intestinale).
